# EUROPEAN PATENT SPECIFICATION

(11) **EP 0 454 795 B1**
(45) Date of publication and mention of the grant of the patent: **20.12.1995**
(21) Application number: 90904584.1
(22) Date of filing: 08.03.1990
(51) Int. Cl.: C12Q 1/00, G01N 33/00, G01N 30/02, G01N 33/546, G01N 33/53

(54) **SCREENING METHOD FOR DIABETIC CONDITION**
VERFAHREN ZUM NACHWEIS DES DIABETISCHEN ZUSTANDES
PROCEDE DE DETECTION D'UN ETAT DIABETIQUE

(30) Priority: 08.03.1989 US 320485
(43) Date of publication of application: 06.11.1991
(73) Proprietor: THE UNIVERSITY OF VIRGINIA PATENTS FOUNDATION, Charlottesville, Virginia 22903 (US)
(72) Inventor: LARNER, Joseph, Charlottesville, VA 22901 (US); KENNINGTON, Alison, Laurel, Maryland 20708 (US); SHEN, Tsung, Y., Charlottesville, VA 22901 (US)
(74) Representative: VOSSIUS & PARTNER
(86) International application number: US9001107
(87) International publication number: WO9010711

(56) References cited:
- Journal of Chromatography, Volume 277 issued 1983, Amsterdam, Netherlands, NIWAet al., "Gas chromatographic-mass spectrometric analysis of polyols in urineand serum of uremic patients: Identification of new deoxyalditols and inositolisomers", pages 25-39: See p25 Summary, p26 lines 11-42, pp. 27-29 "Methods".
- Metabolism-Clinical and Experimental, Volume 35, No. 4, Suppl, issued April,1986, WILLIAMSON et al., "Diabetes-induced increases in vascular permeability and change in granulation tissue levels of sorbitol, myo-inositol, chiro-insitol and scyllo-insitol are prevented by sorbinil", pages 41-45: See p 41 abstract, line 8, p 43 lines 8-19 and Fig. 4, p 44 second col., lines 5-9
- Clinical Chemistry, Volume 30, No. 2 issued 1984, YOSHIOKA et al.,"Concentrations of non-glucose polys in serum and cerebroseinal fluid fromapparently healthy adults and children" pages 188-191.

## Description

This invention pertains to the diabetic condition in mammals, and specifically, a method for screening individual patients for possible diabetes to be confirmed by subsequent analysis. The screening method provides a non-invasive, low-cost, quick method for conducting a preliminary assay for diabetes in mammals, including humans.

Diabetes is perhaps the most common of the serious metabolic diseases of humans. The diabetic condition is a disease prevalent among most mammals. Occasionally, diagnosis of diabetes may be quite simple. If conventional symptoms, attributable to osmotic diuresis are identified, together with hyperglycemia, there is general agreement that the diabetic condition is present. Moreover, persistently elevated fasting plasma glucose concentrations are a strong indication of the diabetic condition, even in the absence of other conventional systems. However, these diagnoses require sophisticated knowledge, on the part of patients, of the appropriate symptoms, or at least persistent medical care and inquiry. Frequently, these are not available.

The alternative, fast and low-cost screening method, the oral glucose tolerance test, is unreliable. Some subjects testing positive, i.e., indicating the diabetic condition, never develop conventional diabetes. Additionally, many patients can move from the non-diabetic condition, and back, merely as a function of weight. Indeed control of obesity in those at risk is a common preventive measure. In addition, the glucose tests are invasive, presenting infection concerns and complications related thereto.

The result is that diabetes remains difficult to accurately diagnose in many cases. As a result, estimates conclude that roughly half of those with type II (insulin-resistant) diabetes in this country have not been diagnosed. At the same time, the oral glucose tolerance test frequently results in false positives, and remains an incomplete screening method.

Accordingly, it remains an object of the art to provide a non-invasive, relatively inexpensive, and quick method for screening potential diabetics in the general population. Such a diagnostic test may not be definitive, but may identify potential candidates for further consideration. Even if not complete, however, a preferred screening method should be relatively insensitive to other conditions which might result in false positives, and not require, at least initially, detailed symptomatic knowledge on the part of the patient.

The above objects, and others that will become apparent upon review of the specification set forth below, are achieved by an assay for the presence of D-chiroinositol in the urine of patients to be screened for the diabetic condition. The urine is simply treated, and of course, can be obtained and analyzed on an out-patient basis. The absence of D-chiroinositol in the urine specimen is a strong indication of the diabetic condition. By "absence", it is indicated that the urine sample contains either no D-chiroinositol, or such extremely low levels as to be effectively absent in vivo.

The diagnostic or screening method addressed herein is based on work reported in U.S. Patent Application Serial No. 07/320,484 (WO-A-90 10641) in the names of Larner, Kennington, Huang and Shen, reporting the purification of at least two insulin mediators. These mediators appear to be formed after the interaction between insulin and the insulin receptor, from precursors which fall into a class of compounds known as glycophospholipid "anchor" substances, being compounds which attach a number of proteins to the cell membrane and apparently aid in transfer of information across the cell membrane into the interior of the cell by interacting with insulin-sensitive enzyme systems. The work reported in U.S. Patent Application Serial No. 07/320,484 (WO-A-90 10641) by Larner et al, confirms the presence in these insulin mediators, of D-chiroinositol.

The diagnostic portion of this invention is based on the observation that the diabetic condition may be associated with chiroinositol. The inventors are unaware of any combination of conditions or states which would permit a patient to avoid diabetes, without the production of D-chiroinositol. Accordingly, absence thereof is a strong indication of the diabetic condition, and can be used as a preliminary screening device, to identify patients who should be studied more rigorously for the possible disease state.

The diabetic condition may be associated with an absence of insulin mediators, or compounds intermediate in the pathway from insulin to the end product or reaction involved. These reactions include the inhibition of cyclic AMP-dependent protein kinase, as well as glucose transport and the stimulation of pyruvate dehydrogenase phosphatase. As the insulin mediators are believed to be produced in response to the presence of insulin, the absence of insulin or the failure to respond to insulin may result in or from an absence of the mediators, thus, an absence of D-chiroinositol may be a strong indication of an absence of insulin mediators from the patient's system.

To screen for the possible diabetic state, a 24-hour urine sample is collected. A fraction of the sample is adsorbed in a mixed anion/cation exchange resin bed, and eluted with distilled, deionized water.

The recovered fraction is further purified with a solid phase extraction on an octadecyl material, such as a C18 SEP-PAK column, available from Waters, Inc.

The sample is selectively eluted with distilled water, the eluant being collected, and lyophilized twice, followed by derivatization.

The derivatized sample is extracted into hexane and subjected to gas chromatography/mass spectrometry.

### CLINICAL EXAMPLE

Twenty 24-hour urine samples were obtained from twenty different human patients. The patients were collected from a pool at the University of Virginia Hospital, Charlottesville, Virginia, as well as the NIH-Diabetes Center, Phoenix, Arizona. Each sample was frozen and stored with 1-3% sodium azide. The storage conditions are necessary to prevent the growth of bacteria. Bacteria in the sample produce D-chiroinositol, which may of course give rise to a false positive. Of course, if the samples are used immediately, no specific storage conditions need be observed. A 10 ml aliquot of each sample was adsorbed onto a mixed anion/cation exchange resin bed and eluted with 5 ml of distilled, deionized water. The recovered fraction was reduced to approximately 5 ml and loaded onto an octadecyl solid phase extraction column, such as the C18 SEP-PAK cartridge, obtained from Waters, Inc. and again eluted with deionised, distilled water. The collected fraction has a light yellow color.

Each fraction was lyophilized twice, and derivatized with heptafluorobutyrlimidazole for six hours at 50°C and twelve hours at ambient temperatures.

The derivatized samples were extracted into hexane, and submitted to gas chromatography/mass spectrometric analysis. Of the twenty samples, one false negative, i.e., an indication of the absence of the diabetic condition, when in fact the patient was diabetic, was detected. In addition, the screening procedure gave three false positives, that is diabetes was indicated (an absence of D-chiroinositol) where the patient was not in fact symptomatically diabetic. The single false negative, and the three false positives were all taken from the samples obtained from the NIH-Diabetic Center, which was drawn from a relatively closed community of American Indians. It is believed that the false readings may be due to physiognomic anomalies due to the sampling of an extremely small gene pool.

### ADDITIONAL EXAMPLE

Repeated testing demonstrates a difference in the concentration of D-chiroinositol in the urine of diabetics, and non-diabetics that is at least 3 orders of magnitude, in samples drawn from the general populace. Thus, the drop in D-chiroinositol of diabetics is easily recognized.

### ALTERNATIVE ASSAYS

In the above testing, the presence of D-chiroinositol was assayed for by use of a gas chromatograph/mass spectrometer. This was conveniently practiced in the laboratory, with good sensitivity. However, other assays may be used without departing from the scope of the invention. Alternative assays include paper chromatography employing chiral-sensitive chromatography paper. Such paper is currently available, and the migration rate of D-chiroinositol may be determined.

Alternate assay formats include an enzyme reduction/oxidation potential measurement. Thus, an enzyme specific for D-chiroinositol may be immobilized on an electrode. The electrode is introduced to the sample, across which a current potential is applied. The presence of D-chiroinositol in the sample will result in the enzyme-catalyzed reaction going forward and alter the electrical environment detected by the electrode on which the enzyme is immobilized. The immobilization of the enzyme itself, in e.g., polyvinylpyrrolidone, or other suitable matrix, is conventional and does not constitute an aspect of the invention.

There are, of course, a wide variety of sensitive assays made available by designed antibodies. Thus, an antibody to a D-chiroinositol-ligand compound substance can be prepared and employed in an ELISA or agglutination assay, as are well known in the art, and may be employed in the context of the invention claimed herein.

It should be noted that many diabetics become symptomatic only upon reaching middle age, upon sudden weight gain, etc. The absence of insulin mediators and, therefore, D-chiroinositol, appears to be genetically controlled. It may be, accordingly, possible to detect a genetic predisposition to the development of diabetes in asymptomatic individuals by use of the assay disclosed herein.

The above invention has been described with reference to specific examples and materials. It should be clear that these specifics can be varied without departing from the scope of the invention. In particular, alternative resins in the purification format, alteration of the purification sequence, and alternate assay forms, sensitive to D-chiroinositol may be employed, without departing from the scope of the invention, as embraced by the claims appended hereto.

## Claims

1. A method for screening a mammalian individual for the possible presence of a Type II diabetic state comprising the steps of:
(a) collecting a urine or serum sample from said individual;
(b) assaying said sample for the presence of D-chiro-inositol;
(c) comparing the concentration of D-chiro-inositol in the sample with a concentration which is characteristic of Type II diabetes, and
(d) classifying the individual as having Type II diabetes if the concentration of D-chiro-inositol in the sample is less than or equal to the concentration which is characteristic of Type II diabetes.

2. The method of claim 1, wherein a concentration of D-chiro-inositol in said sample that is at least 3 orders of magnitude below the concentration in samples drawn from the general populace is indicative of a Type II diabetic state.

3. A method for screening mammalian individuals for a predisposition to Type II diabetes comprising the steps of:
(a) collecting a urine or serum sample from the individual;
(b) assaying said sample for the presence of D-chiro-inositol;
(c) comparing the concentration of D-chiro-inositol in the sample with a concentration which is characteristic of a predisposition to Type II diabetes, and
(d) classifying the individual as having a predisposition to Type II diabetes if the concentration of D-chiro-inositol in the sample is less than or equal to the concentration which is characteristic of a predisposition to Type II diabetes.

4. The method of claim 3, wherein a concentration of D-chiro-inositol in said sample that is at least 3 orders of magnitude below the concentration in samples drawn from the general populace is indicative of a predisposition to Type II diabetes.

5. The method of any one of claims 1 to 4, wherein said sample is a 24-hour urine sample.

6. The method of any one of claims 1 to 5, wherein said individual is a human.

7. The method of any one of claims 1 to 6, further comprising the step of purifying said sample after step (a).

8. The method of claim 7, wherein said sample is purified by adsorption onto, and elution from a mixed anion/cation exchange resin chromatographic column and an octadecyl solid phase extraction column in sequence, followed by lyophilization and derivatization.

9. The method of any one of claims 1 to 8, wherein step (b) is practiced using an assay selected from the group consisting of gas chromatographic/mass spectrometric analysis, paper chromatography, enzyme specific reduction/oxidation potential analysis, wherein said enzyme is specific for D-chiro-inositol and an ELISA assay or agglutination assay employing an antibody to a D-chiro-inositol ligand compound substance.

## Patentansprüche

1. Verfahren zum Screenen eines Säugerindividuums auf das mögliche Vorhandensein eines Typ II-Diabeteszustands, umfassend die folgenden Schritte:
(a) Gewinnung einer Urin- oder Serumprobe von diesem Individuum,
(b) Testen der Probe auf die Gegenwart von D-Chiroinosit,
(c) Vergleich der Konzentration von D-Chiroinosit in der Probe mit einer Konzentration, die charakteristisch für Typ II-Diabetes ist, und
(d) Klassifizierung des Individuums als Träger von Typ II-Diabetes, wenn die Konzentration von D-Chiroinosit in der Probe geringer ist als oder gleich der Konzentration, die charakteristisch für Typ II-Diabetes ist.

2. Verfahren nach Anspruch 1, wobei eine Konzentration von D-Chiroinosit in der Probe, die mindestens drei Größenordnungen unter der Konzentration in Proben liegt, die von der Allgemeinbevölkerung gewonnen wurden, auf einen Typ II-Diabeteszustand hinweist.

3. Verfahren zum Screenen von Säugerindividuen auf eine Predisposition für Typ II-Diabetes, umfassend die folgenden Schritte:
(a) Gewinnung einer Urin- oder Serumprobe von dem Individuum,
(b) Testen der Probe auf die Gegenwart von D-Chiroinosit,
(c) Vergleich der Konzentration von D-Chiroinosit in der Probe mit einer Konzentration, die charakteristisch ist für eine Predisposition für Typ II-Diabetes, und
(d) Klassifizierung des Individuums als Träger einer Predisposition für Typ II-Diabetes, wenn die Konzentration von D-Chiroinosit in der Probe geringer ist als oder gleich der Konzentration, die charakteristisch ist für eine Predisposition für Typ II-Diabetes.

4. Verfahren nach Anspruch 3, wobei eine Konzentration von D-Chiroinosit in der Probe, die mindestens drei Größenordnungen unter der Konzentration in Proben liegt, die von der Allgemeinbevölkerung gewonnen wurden, auf eine Predisposition für Typ II-Diabetes hinweist.

5. Verfahren nach einem der Ansprüche 1 bis 4, wobei die Probe eine 24 Stunden-Urinprobe ist.

6. Verfahren nach einem der Ansprüche 1 bis 5, wobei das Individuum ein Mensch ist.

7. Verfahren nach einem der Ansprüche 1 bis 6, das als weiteren Schritt die Reinigung der Probe nach Schritt (a) umfaßt.

8. Verfahren nach Anspruch 7, wobei die Probe durch Adsorption an und Elution von einer Chromatographiesäule mit einem gemischten Anionen/Kationenaustauscherharz und einer anschließenden Octadecyl-Festphasenextraktionssäule gereinigt wird, gefolgt von Gefriertrocknung und Derivatisierung.

9. Verfahren nach einem der Ansprüche 1 bis 8, wobei Schritt (b) unter Verwendung eines Tests durchgeführt wird, der ausgewählt ist aus Gaschromatographie/Massenspektrometrieaanalyse, Papierchromatographie, enzymspezifisches Reduktions/Oxidationspotential-Analyse, wobei das Enzym spezifisch ist für D-Chiroinosit, und einem ELISA-Test oder Agglutinationstest unter Verwendung eines Antikörpers gegen eine D-Chiroinosit-Ligandenverbindungssubstanz.

## Revendications

1. Procédé de dépistage chez un individu mammalien de la présence possible d'un état diabétique du type II, comprenant les étapes consistant à :
(a) recueillir un échantillon d'urine ou de sérum chez ledit individu;
(b) rechercher dans ledit échantillon la présence de D-chiro-inositol;
(c) comparer la concentration de D-chiro-inositol dans l'échantillon avec une concentration caractéristique du diabète de type II et
(d) classer l'individu comme ayant un diabète de type II si la concentration de D-chiro-inositol dans l'échantillon est inférieure ou égale à la concentration caractéristique du diabète de type II.

2. Procédé selon la revendication 1, dans lequel une concentration de D-chiro-inositol dans ledit échantillon inférieure d'au moins trois ordres de grandeur à la concentration dans des échantillons prélevés sur la population générale témoigne d'un état diabétique de type II.

3. Procédé de dépistage chez des individus mammaliens d'une prédisposition au diabète de type II comprenant les étapes consistant à :
(a) recueillir un échantillon d'urine ou de sérum chez l'individu;
(b) rechercher dans ledit échantillon la présence de D-chiro-inositol;
(c) comparer la concentration de D-chiro-inositol dans l'échantillon avec une concentration caractéristique d'une prédisposition au diabète de type II et
(d) classer l'individu comme ayant une prédisposition au diabète de type II si la concentration de D-chiro-inositol dans l'échantillon est inférieure ou égale à la concentration caractéristique d'une prédisposition au diabète de type II.

4. Procédé selon la revendication 3, dans lequel une concentration de D-chiro-inositol dans ledit échantillon inférieure d'au moins trois ordres de grandeur à la concentration dans des échantillons prélevés sur la population générale témoigne d'une prédisposition au diabète de type II.

5. Procédé selon l'une quelconque des revendications 1 à 4, dans lequel ledit échantillon est un échantillon d'urine de 24 h.

6. Procédé selon l'une quelconque des revendications 1 à 5, dans lequel ledit individu est un sujet humain.

7. Procédé selon l'une quelconque des revendications 1 à 6, comprenant en outre l'étape consistant à purifier ledit échantillon après l'étape (a).

8. Procédé selon la revendication 7, dans lequel ledit échantillon est purifié par adsorption et élution sur successivement une colonne de chromatographie à résine mixte échangeuse d'anions/cations et une colonne d'extraction en phase solide octadécylique, suivies d'une lyophilisation et d'une dérivation.

9. Procédé selon l'une quelconque des revendications 1 à 8, dans lequel l'étape (b) est mise en oeuvre à l'aide d'un dosage sélectionné dans le groupe constitué par l'analyse par chromatographie en phase gazeuse/spectrométrie de masse, la chromatographie sur papier, l'analyse du potentiel de réduction/oxydation enzymatique spécifique où ladite enzyme est spécifique du D-chiro-inositol et un dosage ELISA ou un dosage par agglutination utilisant un anticorps dirigé contre une substance composite D-chiro-inositol-ligand.
